# EUROPÄISCHE PATENTSCHRIFT

(11) **EP 1 886 018 B1**
(45) Veröffentlichungstag und Bekanntmachung des Hinweises auf die Patenterteilung: **07.09.2011**
(21) Anmeldenummer: 06753930.4
(22) Anmeldetag: 26.05.2006
(51) Int. Cl.: F04B 13/00, F04B 43/02, A61M 1/16, F04B 49/06

(54) **VORRICHTUNG UND VERFAHREN ZUR FÖRDERUNG MEDIZINISCHER FLÜSSIGKEITEN**
DEVICE AND METHOD FOR TRANSPORTING MEDICINAL LIQUIDS
DISPOSITIF ET PROCEDE DE TRANSPORT DE LIQUIDES MEDICAUX

(30) Priorität: 27.05.2005 DE 102005024363
(43) Veröffentlichungstag der Anmeldung: 13.02.2008
(73) Patentinhaber: Fresenius Medical Care Deutschland GmbH, 61352 Bad Homburg v.d.H. (DE)
(72) Erfinder: IMHOF, Peter, 97422 Schweinfurt (DE); GAGEL, Alfred, 96123 Litzendorf (DE)
(74) Vertreter: Herrmann, Uwe
(86) Internationale Anmeldenummer: PCT/EP2006/005080
(87) Internationale Veröffentlichungsnummer: WO 2006/125671

(56) Entgegenhaltungen:
- EP-A- 0 160 272
- WO-A-03/054392
- DE-A1- 10 108 506
- US-A- 5 431 634
- US-A- 6 065 940
- US-A1- 2003 220 609

## Beschreibung

Die Erfindung betrifft ein Dialysegerät mit einer Vorrichtung zur Förderung von Konzentrat, das zur Zubereitung von Dialyseflüssigkeiten in einem Dialysegerät dient, nach dem Oberbegriff von Anspruch 1, sowie ein Verfahren zur Förderung solch eines Konzentrats nach dem Oberbegriff von Anspruch 11.

Der Einsatz von Verdrängerpumpen ist in zahlreichen unterschiedlichen Anwendungsgebieten, wie z. B. im Bereich der Medizintechnik bekannt. Es sind beispielsweise Dialysegeräte bekannt, bei denen Dialysierflüssigkeit unter Zuhilfenahme von Verdrängerpumpen hergestellt wird. In derartigen Dialysegeräten wird die Dialysierflüssigkeit aus Reinstwasser (RO-Wasser) sowie aus einem oder mehreren Konzentraten hergestellt, die die benötigten Elektrolyte und andere Inhaltsstoffe enthalten. Das Reinstwasser sowie die Konzentrate werden in einem geeigneten Verhältnis gemischt. Das Mischungsverhältnis zwischen Reinstwasser und Konzentrat kann beispielsweise bei 34:1 oder 44:1 liegen. Selbstverständlich sind auch davon abweichende Verhältnisse denkbar.

Die Konzentratförderung wird häufig mittels Membranpumpen durchgeführt, die mit definiertem Hubvolumen arbeiten. Ein Dialysegerät mit einer Membranpumpe zur Förderung des Konzentrates ist aus der DE 28 38 414 bekannt. Bei dem aus dieser Druckschrift bekannten Gerät wird Reinstwasser nach Passieren eines Druckminderventils mit Konzentrat gemischt. Die Förderung des Konzentrates erfolgt synchron mit der Betätigung der Bilanziervorrichtung, so dass pro Arbeitstakt der Bilanziervorrichtung jeweils ein Arbeitstakt oder eine definierte Anzahl von Arbeitstakten der Pumpe vorgenommen wird.

Bei vorbekannten Dialysegeräten wird die Dialysierflüssigkeit häufig in diskreten Portionen, d.h. in Form sogenannter Batches zusammengestellt. Die Menge des zu fördernden Konzentrates ist nicht konstant, sondern kann von Konzentrat zu Konzentrat bzw. in Abhängigkeit der vorzunehmenden Behandlung variieren. Bei verschiedenen Konzentraten tritt somit häufig der Fall ein, dass diese nicht alle in derselben Menge gefördert werden müssen, sondern dass sich die Fördervolumina erheblich voneinander unterscheiden können. Denkbar ist beispielsweise, dass sich der Bereich der Fördervolumina bei einem Batchvolumen von 30 ml von 200 µl bis 2200 µl erstreckt.

Bei Verdrängerpumpen ergibt sich das Problem, dass die Genauigkeit der Förderung von dem Fördervolumen abhängt. Eine besonders hohe Genauigkeit ergibt sich dann, wenn für einen Batch ein Hub mit einem Fördervolumen verwendet wird, das dem maximal möglichen Fördervolumen pro Hub entspricht oder diesem zumindest angenähert ist.

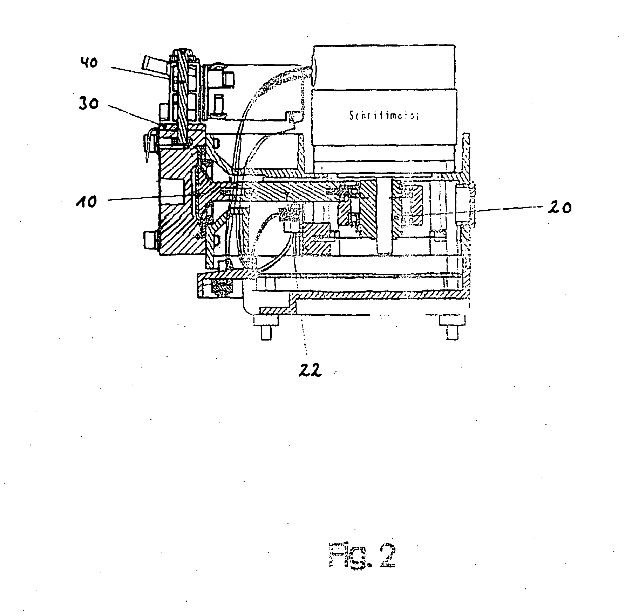

Ein Ausführungsbeispiel für eine aus dem Stand der Technik bekannte Membranpumpe ist aus Fig. 2 im Längsschnitt ersichtlich. Die Pumpe weist eine Membran 10 auf, die den Förderraum der Pumpe auf einer Seite begrenzt und deren Bewegung zu einer Förderung von Flüssigkeit führt. Die Bewegung der Membran 10 wird durch einen Exzenterantrieb 20 mit Schrittmotor realisiert. Die Pleuelstange 22 steht mit der Membran 10 in Verbindung und setzt die Bewegung des Exzenterantriebs 20 in eine Bewegung der Membran 10 um. Das Bezugszeichen 30 zeigt das Pumpengehäuse, das sich aus dem Antriebsgehäuse und dem mit diesem verschraubten Pumpenkopf zusammensetzt, in dem auch die Ventile angeordnet sind, die die Befüllung und Entleerung des Förderraums steuern. Eines der Ventile ist in Fig. 2 mit dem Bezugszeichen 40 gekennzeichnet.

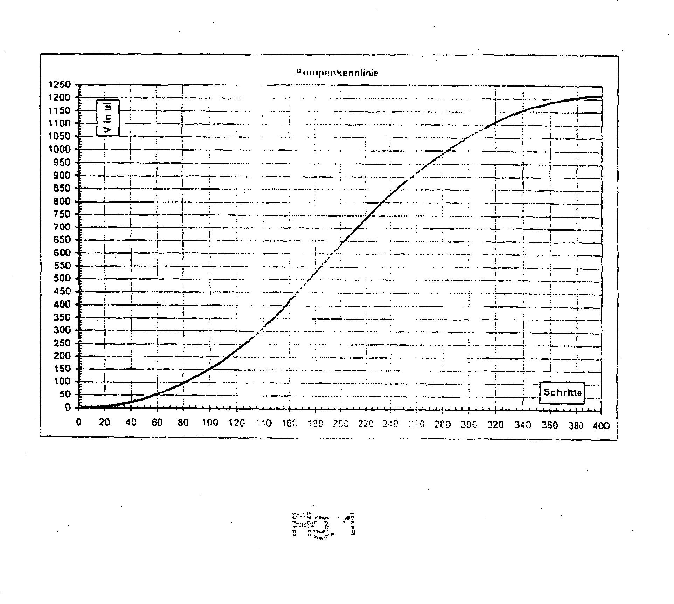

Fig. 1 zeigt eine Pumpenkennlinie der Pumpe gemäß Fig. 2. In dem hier dargestellten Ausführungsbeispiel ist die Pumpe so dimensioniert, dass sie pro Hub etwa maximal 1200 µl fördern kann. Bei dieser Pumpe ist die geforderte Genauigkeit von 1 % ab Fördervolumina von ca. 600 µl durch eine individuelle Kalibrierung der Pumpe gut realisierbar. Hinsichtlich der Genauigkeit stellt sich jedoch der untere Bereich von 200 µl bis 600 µl als problematischer dar, wobei insbesondere der Bereich zwischen 200 µl und 300 µl für die Anwendung von Trockenkonzentrat wichtig ist. Aufgrund der Abhängigkeit vom ein- und ausgangsseitigen Druck sowie der vorhandenen zeitlichen Drift kann in diesem Bereich die Genauigkeit auch nicht durch eine bessere Kalibrierung erreicht werden.

Somit ergibt sich das Problem, dass zur Gewährleistung der geforderten Genauigkeit insbesondere für kleine Fördervolumina spezielle Pumpen erforderlich wären, was mit dem Nachteil eines vergleichsweise hohen apparativen Aufwandes verbunden wäre.

Ein Dialysegerät mit mehreren Konzentratpumpen ist beispielsweise aus der EP 160 272 bekannt, wie es im Oberbegriff von Anspruch 1 definiert ist. Eine der Konzentratpumpen ist dafür vorgesehen, eine Basisdialysierflüssigkeit bereitzustellen, deren Ionenkonzentration für die Behandlung unbedingt erforderlich ist. Die zweite Konzentratpumpe bewirkt die Anpassung der Ionenkonzentration der Dialysierflüssigkeit an die individuellen Bedürfnisse, die von Patient zu Patient variieren können.

Aus der WO 99/30756 ist eine Verdrängerpumpe eines Dialysegerätes bekannt, die aus einem Behältnis Konzentrat zu einem Mischpunkt fördert. Sobald in dem Behältnis ein bestimmter Füllstand unterschritten ist, wird das Behältnis durch eine baugleiche Förderpumpe mit Konzentrat über den genannten Füllstand hinaus gefüllt. Auf diese Weise wird sichergestellt, dass bei Betrieb der das Behältnis leerenden Pumpe stets ein Signal abgegeben wird, das dann erzeugt wird, wenn der genannte Füllstand unterschritten wird. Das Signal aktiviert die das Behältnis füllende Pumpe.

Es ist die Aufgabe der vorliegenden Erfindung eine Vorrichtung der eingangs genannten Art dahingehend weiterzubilden, dass mit geringem apparativen Aufwand eine genaue Dosierung der Flüssigkeit auch bei kleineren Fördervolumina ermöglicht wird.

Diese Aufgabe wird durch ein Dialysegerät mit den Merkmalen des Anspruchs 1 gelöst, sowie durch ein Verfahren mit den Merkmalen des Anspruchs 11. Danach ist vorgesehen, dass die Pumpe durch eine Steuereinheit derart ansteuerbar ist, dass die Pumpe zum Zwecke der Abgabe eines Sollfördervolumens in einem ersten Betriebszustand in einem einzigen Saughub ein das Sollfördervolumen übersteigendes Volumen ansaugt und das angesaugte Volumen durch Vornahme von mehreren Teilförderhüben in Form von Teilvolumina portionsweise abgibt, die kleiner sind als das angesaugte Volumen. Die Erfindung besteht somit darin, dass, um ein bestimmtes Volumen zu fördern, in einem Saughub ein größeres Volumen angesaugt wird. Dieses Volumen wird nach dem Ansaugvorgang vorzugsweise durch Schließen des Eingangsventils begrenzt. Aufgrund des vergleichsweise großen angesaugten Volumens ergibt sich eine hohe Genauigkeit. Das angesaugte Volumen wird nach Abschluss des Saughubes portionsweise abgeben, wobei vorzugsweise angestrebt wird, dass die Teilvolumina wenigstens in etwa den Sollfördervolumina entsprechen.

Systematische Unterschiede in den Teilvolumina können gegebenenfalls durch eine Pumpensoftware korrigiert werden. Auch eine zusätzlich Kalibrierung ist vorstellbar, um die Genauigkeit der Teilvolumina zu verbessern.

Wesentlicher Vorteil der Erfindung ist es, dass die Teilvolumina im Mittel mit hoher Genauigkeit den Sollfördervolumina angenähert sind oder diesen im Idealfall entsprechen.

Weitere Vorteile der Erfindung sind Gegenstand der Unteransprüche.

Bei der Pumpe kann es sich um eine beliebige Verdrängerpumpe, wie z.B. um eine Membran- oder Kolbenpumpe handeln.

Besonders vorteilhaft ist es, wenn die Pumpe durch die Steuereinheit derart ansteuerbar ist, dass das in einem Saughub angesaugte, das Sollfördervolumen übersteigende Volumen in einem Bereich von 0,5 bis 1,0 des maximalen Fördervolumens pro Hub der Pumpe liegt. Wie oben ausgeführt, ergibt sich eine besonders hohe Genauigkeit, wenn ein Volumen angesaugt wird, das möglichst dicht an dem maximalen Fördervolumen liegt. In einer weiteren Ausgestaltung der Erfindung ist daher vorgesehen, dass die Pumpe durch die Steuereinheit derart ansteuerbar ist, dass das in einem Saughub angesaugte, das Sollfördervolumen übersteigende Volumen dem maximalen Fördervolumen der Pumpe wenigstens annähernd entspricht.

Sollen gleiche oder im wesentlichen gleiche Teilvolumina bereitgestellt werden, wie dies beispielsweise dann der Fall sein kann, wenn die Pumpe zur Förderung von Konzentrat zur Zubereitung einer Dialysierflüssigkeit dient, kann vorgesehen sein, dass die Pumpe durch die Steuereinheit derart ansteuerbar ist, dass die abgegebenen Teilvolumina identisch oder im wesentlichen identisch sind.

Das Verhältnis aus dem in einem Saughub angesaugtem Volumen und dem pro Teilhub abgegebenen Teilvolumen ist beliebig. In einer Ausgestaltung ist vorgesehen, dass das Verhältnis aus dem angesaugten Volumen und einem abgegebenen Teilvolumen bei 10:1 oder darunter liegt. Denkbar ist somit beispielsweise, dass ein Teilvolumen die Hälfte oder ein Drittel des angesaugten Volumens beträgt, d.h. das zwei bzw. drei Teilförderhübe erfolgen, bevor der nächste Saughub durchgeführt wird.

Die erfindungsgemäße Vorrichtung wird in einer Ausführungsform der Erfindung stets in dem genannten ersten Betriebszustand betrieben, in dem das in einem Saughub angesaugte Volumen auf mehrere Teilvolumina aufgeteilt wird. Denkbar ist ferner eine Ausgestaltung der Erfindung, bei der die Pumpe in einem zweiten Betriebszustand betreibbar ist, in dem keine Aufteilung des angesaugten Volumens in Teilvolumina erfolgt, sondern das in einem Saughub aufgenommene Volumen in einem Förderhub abgegeben wird.

Dabei kann vorgesehen sein, dass die Pumpe von der Steuereinheit derart ansteuerbar ist, dass die Wahl des Betriebszustandes durch die Steuereinheit von dem Sollfördervolumen abhängt.

Wie oben ausgeführt, können sich Ungenauigkeiten bei der Förderung insbesondere dann ergeben, wenn verhältnismäßig kleine Volumina gefördert werden sollen. Daher besteht eine weitere Ausgestaltung der Erfindung darin, dass die Pumpe von der Steuereinheit derart ansteuerbar ist, dass der erste Betriebszustand bei kleineren Sollfördervolumina und der zweite Betriebszustand, bei dem das angesaugte Volumen in einem Förderhub abgegeben wird, bei demgegenüber größeren Sollfördervolumina eingestellt wird.

Denkbar ist, dass die Pumpe von der Steuereinheit derart ansteuerbar ist, dass der erste Betriebszustand eingestellt wird, wenn das Sollfördervolumen maximal die Hälfte, ein Drittel oder ein Viertel des maximalen Fördervolumens pro Hub der Pumpe beträgt.

Die Erfindung betrifft ferner die Verwendung einer Vorrichtung nach einem der Ansprüche 1 bis 10 zur Förderung eines Konzentrates, das zur Zubereitung von Dialysierflüssigkeiten dient.

Denkbar ist, dass die Dialysierflüssigkeit in einem Dialysegerät zubereitet wird, wobei die erfindungsgemäße Vorrichtung Bestandteil des Dialysegerätes ist. Alternativ dazu ist möglich, dass die Vorrichtung nicht Bestandteil des Dialysegerätes ist und dass mittels der Vorrichtung das Konzentrat oder eine mit dem Konzentrat gebildete Dialysierflüssigkeit zu dem Dialysegerät gefördert wird.

Die Erfindung betrifft ferner ein Dialysegerät mit einer Vorrichtung nach einem der Ansprüche 1 bis 10. Wie oben ausgeführt, kommt die erfindungsgemäße Vorrichtung insbesondere dann zum Einsatz, wenn in wenigstens einem Betriebszustand im Batch-Betrieb gearbeitet wird. Im Falle der Verwendung der Vorrichtung in einem Dialysegerät erweist es sich als vorteilhaft, dass es in diesem Fall häufig nicht entscheidend ist, dass jeder Batch die exakte Dosierung enthält. Von der Mischstelle des Konzentrats mit dem RO-Wasser bis zum Dialysator sind weitere Bauteile, wie beispielsweise die Mischkammer, Bilanzkammer, Sterilfilter und der Dialysator selbst vorhanden, in deren Volumina sich die einzelnen Batches vermischen können. Darüber hinaus sind kleinere Leitfähigkeitsschwankungen tolerierbar. Entscheidend ist, dass im Mittel über eine bestimmte Anzahl (z.B. 4) Batches eine exakte Dosierung gesichert ist, wie dies vorliegend der Fall ist.

Das erfindungsgemäße Dialysegerät kann ein Konzentratbehältnis zur Aufnahme von Konzentrat zur Zubereitung von Dialysierflüssigkeiten aufweisen, wobei die Verdrängerpumpe der Vorrichtung saugseitig mit dem Konzentratbehältnis in Verbindung steht. Druckseitig ist vorzugsweise ein Mischpunkt vorgesehen, in dem das geförderte Konzentrat mit Reinstwasser gemischt wird.

Die Erfindung betrifft ferner ein Verfahren zur Förderung einer Flüssigkeit, vorzugsweise einer medizinischen Flüssigkeit, bei dem die Flüssigkeit durch eine als Verdrängerpumpe ausgeführte Pumpe gefördert wird, wobei die Pumpe in einem ersten Betriebszustand in einem Saughub ein das Sollfördervolumen übersteigendes Volumen ansaugt und das angesaugte Volumen durch Vornahme von mehreren Teilförderhüben in Form von Teilvolumina portionsweise abgibt, die kleiner sind als das angesaugte Volumen.

Vorteilhafte Ausgestaltungen des Verfahrens sind Gegenstand der Unteransprüche. Besonders vorteilhaft ist es, wenn das in einem Saughub angesaugte, das Sollfördervolumen übersteigende Volumen dem maximalen Fördervolumen pro Hub der Pumpe entspricht oder diesem möglichst nahe kommt.

Weitere Einzelheiten und Vorteile der Erfindung werden anhand eines im Folgenden dargestellten Ausführungsbeispiels näher erläutert. Es zeigen:
Fig. 1: eine Pumpenkennlinie (Fördervolumen vs. Schrittzahl) einer Membranpumpe;
Fig. 2: eine Schnittansicht einer Membranpumpe und
Fig. 3: eine Fehlerkennlinie unter Anwendung der Erfindung im Bereich zwischen 200 µl und 300 µl.

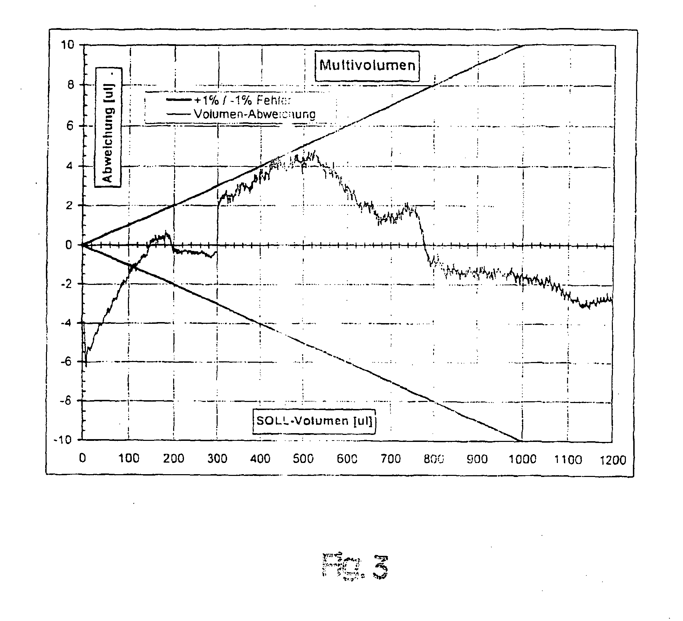

Fig. 3 zeigt eine Fehlerkennlinie einer Membranpumpe, die ein maximales Fördervolumen von 1200 µl ml pro Hub aufweist. Der Kurvenverlauf zeigt die Abweichung in µl des geförderten Volumens vom Sollfördervolumen in Abhängigkeit vom Sollfördervolumen in µl. Die Fehlergeraden zeigen die 1 % - Abweichung des Fördervolumens vom Sollfördervolumen sowohl bei Abweichungen nach oben ( + 1 %) als auch nach unten (- 1 %).

Aus Fig. 3 ist erkennbar, dass bei größeren Sollfördervolumina die Abweichung des Istwertes vom Sollwert vergleichsweise gering ist, die Genauigkeit der Förderung somit zufriedenstellend ist.

Fig. 3 zeigt weiter, dass sich eine ungenauere Förderung insbesondere im Bereich unterhalb von 600 µl ergibt. Die hier erreichten Genauigkeiten bewegen sich im Bereich der + 1 % -Fehlergeraden.

In dem Bereich unterhalb des Sollfördervolumens von 200 µl und oberhalb von 300 µl wurde das angesaugte Volumen in einem einzigen Förderhub abgegeben, was dem oben erwähnten zweiten Betriebszustand der Pumpe entspricht. Im Bereich zwischen 200 µl und 300 µl wurde die Pumpe in dem ersten Betriebszustand betrieben, d.h. in diesem Bereich wurde das erfindungsgemäße Förderprinzip angewandt. Der in Fig. 3 angegebene Begriff "Multivolumen" verdeutlicht, dass in dem genannten Bereich zwischen 200 µl und 300 µl zur Abgabe des Sollfördervolumens nicht dieser Wert, sondern ein größeres Volumen, beispielsweise 1200 µl in einem Saughub angesogen wurde, das sodann in Form mehrerer Teilvolumina abgegeben wurde. Zur Förderung im Bereich zwischen 200 µl und 300 µl wurde jeweils das vierfache Volumen angesaugt und in vier Teilvolumina gefördert.

Denkbar ist selbstverständlich auch eine Vorgehensweise, bei der in dem Bereich bis 300 µl in Abhängigkeit des Sollfördervolumens eine veränderliche Anzahl von Teilvolumina gefördert wird. Denkbar ist, dass bei Sollfördervolumina bis 200 µl das Sechsfache des Sollfördervolumens in einem Saughub angesaugt wird und das angesaugte Volumen in Form von sechs Teilvolumina abgegeben wird. In dem Bereich zwischen 201 µl und 240 µl kann vorgesehen sein, dass das Fünffache des Sollfördervolumens in einem Saughub angesaugt wird und das angesaugte Volumen in Form von fünf Teilvolumina abgegeben wird. Zwischen 241 µl und 300 µl kann vorgesehen sein, dass das Vierfache des Sollfördervolumens in einem Saughub angesaugt wird und das angesaugte Volumen in Form von vier Teilvolumina abgegeben wird.

Eine derartige Abstufung kann auch über den Bereich von 300 µl hinaus vorgesehen sein. Denkbar ist, dass in dem Intervall von 301 µl bis 400 µl das dreifache Volumen und in dem Bereich von 401 µl bis 600 µl das zweifache Volumen angesaugt und in Form von drei bzw. zwei Teilvolumina abgegeben wird. Ab 601 µl kann vorgesehen sein, dass das angesaugte Volumen dem Sollfördervolumen entspricht und dass das angesaugte Volumen in einem Förderhub abgegeben wird.

Denkbar ist selbstverständlich auch, dass bereits ab 301 µl das angesaugte Volumen dem Sollfördervolumen entspricht und dass das angesaugte Volumen in einem Förderhub abgegeben wird.

Aufgrund des vergleichsweise großen angesaugten Volumens ergibt sich in dem "Multivolumen-Bereich" zwischen 200 µl und 300 µl eine hervorragende Fördergenauigkeit, wie dies in Fig. 3 dadurch ersichtlich ist, dass die auf der Ordinate aufgetragene Abweichung zwischen Soll- und Istwert sehr gering ist. Damit ergibt sich der Vorteil, dass für die Förderung unterschiedlicher Volumina und somit auch für die Förderung unterschiedlicher Konzentrate eine einzige Pumpe verwendet werden kann, wobei für den gesamten Sollförderbereich eine sehr gute Genauigkeit erzielbar ist.

## Patentansprüche

1. Dialysegerät mit einer Vorrichtung zur Förderung von Konzentrat, das zur Zubereitung von Dialysierflüssigkeiten in einem Dialysegerät dient, wobei die Vorrichtung wenigstens eine als Verdrängerpumpe ausgebildete Pumpe sowie wenigstens eine den Betrieb der Pumpe steuernde Steuereinheit aufweist,
**dadurch gekennzeichnet,**
**dass** die Steuereinheit vorgibt, dass die Pumpe zum Zwecke der Abgabe eines Sollfördervolumens in einem ersten Betriebszustand in einem einzigen Saughub ein das Sollfördervolumen übersteigendes Volumen ansaugt und das angesaugte Volumen durch Vornahme von mehreren Teilförderhüben in Form von Teilvolumina portionsweise abgibt, die kleiner sind als das angesaugte Volumen, wobei die Teilvolumina jeweils dem Sollfördervolumen entsprechen.

2. Dialysegerät nach Anspruch 1, **dadurch gekennzeichnet, dass** es sich bei der Pumpe um eine Membran- oder Kolbenpumpe handelt.

3. Dialysegerät nach Anspruch 1 oder 2, **dadurch gekennzeichnet, dass** die Steuereinheit vorgibt, dass das in einem Saughub angesaugte, das Sollfördervolumen übersteigende Volumen in einem Bereich von 0,5 bis 1,0 des maximalen Fördervolumens pro Hub der Pumpe liegt.

4. Dialysegerät nach einem der vorhergehenden Ansprüche, **dadurch gekennzeichnet, dass** die Steuereinheit vorgibt, dass das in einem Saughub angesaugte, das Sollfördervolumen übersteigende Volumen dem maximalen Fördervolumen pro Hub der Pumpe entspricht.

5. Dialysegerät nach einem der vorhergehenden Ansprüche, **dadurch gekennzeichnet, dass** die Steuereinheit vorgibt, dass die abgegebenen Teilvolumina identisch oder im wesentlichen identisch sind.

6. Dialysegerät nach einem der vorhergehenden Ansprüche, **dadurch gekennzeichnet, dass** das Verhältnis aus dem in einem Saughub angesaugten Volumen und einem abgegebenen Teilvolumen bei 10:1 oder darunter liegt.

7. Dialysegerät nach einem der vorhergehenden Ansprüche, **dadurch gekennzeichnet, dass** die Steuereinheit abhängig von dem Sollfördervolumen den ersten oder einen zweiten Betriebszustand der Pumpe vorgibt, in dem keine Aufteilung des in einem Saughub angesaugten Volumens in Teilvolumina erfolgt.

8. Dialysegerät nach Anspruch 7, **dadurch gekennzeichnet, dass** der erste Betriebszustand bei kleineren Sollfördervolumina und der zweite Betriebszustand bei demgegenüber größeren Sollfördervolumina eingestellt wird.

9. Dialysegerät nach Anspruch 8, **dadurch gekennzeichnet, dass** der erste Betriebszustand eingestellt wird, wenn das Sollfördervolumen maximal die Hälfte, ein Drittel oder ein Viertel des maximalen Fördervolumens pro Hub der Pumpe beträgt.

10. Dialysegerät nach einem der vorhergehenden Ansprüche, **dadurch gekennzeichnet, dass** das Dialysegerät ein Konzentratbehältnis zur Aufnahme des Konzentrats aufweist und dass die Pumpe der Vorrichtung saugseitig mit dem Konzentratbehältnis in Verbindung steht.

11. Verfahren zur Förderung von Konzentrat, das zur Zubereitung von Dialysierflüssigkeiten in einem Dialysegerät dient, wobei das Konzentrat durch eine als Verdrängerpumpe ausgeführte Pumpe gefördert wird,
**dadurch gekennzeichnet,**
**dass** die Pumpe in einem ersten Betriebszustand zum Zwecke der Abgabe eines Sollfördervolumens in einem einzigen Saughub ein das Sollfördervolumen übersteigendes Volumen ansaugt und das angesaugte Volumen durch Vornahme von mehreren Teilförderhüben in Form von Teilvolumina portionsweise abgibt, die kleiner sind als das angesaugte Volumen, wobei die Teilvolumina jeweils dem Sollfördervolumen entsprechen.

12. Verfahren nach Anspruch 11, **dadurch gekennzeichnet, dass** das in einem Saughub angesaugte, das Sollfördervolumen übersteigende Volumen in einem Bereich von 0,5 bis 1,0 des maximalen Fördervolumens pro Hub der Pumpe liegt.

13. Verfahren nach Anspruch 11 oder 12, **dadurch gekennzeichnet, dass** das in einem Saughub angesaugte, das Sollfördervolumen übersteigende Volumen dem maximalen Fördervolumen pro Hub der Pumpe entspricht.

14. Verfahren nach einem der Ansprüche 11 bis 13, **dadurch gekennzeichnet, dass** die abgegebenen Teilvolumina identisch oder im wesentlichen identisch sind.

15. Verfahren nach einem der Ansprüche 11 bis 14, **dadurch gekennzeichnet, dass** das Verhältnis aus dem in einem Saughub angesaugten Volumen und einem abgegebenen Teilvolumen bei 10:1 oder darunter liegt.

16. Verfahren nach einem der Ansprüche 11 bis 15, **dadurch gekennzeichnet, dass** die Pumpe in einem zweiten Betriebszustand das in einem Saughub angesaugte Volumen in einem Förderhub abgibt.

17. Verfahren nach Anspruch 16 sowie nach einem der Ansprüche 11 bis 15, **dadurch gekennzeichnet, dass** der Betriebszustand der Pumpe in Abhängigkeit des Sollfördervolumens gewählt wird.

18. Verfahren nach Anspruch 17, **dadurch gekennzeichnet, dass** die Pumpe bei kleineren Sollfördervolumina in ihrem ersten Betriebszustand und bei demgegenüber größeren Sollfördervolumina in ihrem zweiten Betriebszustand arbeitet.

19. Verfahren nach Anspruch 18, **dadurch gekennzeichnet, dass** die Pumpe in ihrem ersten Betriebszustand arbeitet, wenn das Sollfördervolumen maximal die Hälfte, ein Drittel oder ein Viertel des maximalen Fördervolumens pro Hub der Pumpe beträgt.

## Claims

1. A dialysis machine with a device for delivering concentrate which serves for preparing dialysis fluids in a dialysis machine, wherein the device includes at least one pump constituting a displacement pump and at least one control unit controlling the operation of the pump,
**characterized in**
**that** the control unit specifies that during a single suction stroke in a first operating condition the pump sucks in a volume exceeding the desired delivery volume for the purpose of discharging a desired delivery volume, and discharges portions of the volume sucked in by performing a plurality of partial delivery strokes in the form of partial volumes which are smaller than the volume sucked in, wherein the partial volumes each correspond to the desired delivery volume.

2. The dialysis machine according to claim 1, **characterized in that** the pump is a diaphragm or piston pump.

3. The dialysis machine according to claim 1 or 2, **characterized in that** the control unit specifies that the volume sucked in during a suction stroke, which exceeds the desired delivery volume, lies in a range from 0.5 to 1.0 of the maximum delivery volume per stroke of the pump.

4. The dialysis machine according to any of the preceding claims, **characterized in that** the control unit specifies that the volume sucked in during a suction stroke, which exceeds the desired delivery volume, corresponds to the maximum delivery volume per stroke of the pump.

5. The dialysis machine according to any of the preceding claims, **characterized in that** the control unit specifies that the partial volumes discharged are identical or substantially identical.

6. The dialysis machine according to any of the preceding claims, **characterized in that** the ratio of the volume sucked in during a suction stroke to a partial volume discharged is about 10:1 or below.

7. The dialysis machine according to any of the preceding claims, **characterized in that** in dependence on the desired delivery volume the control unit specifies the first or a second operating condition of the pump, in which the volume sucked in during a suction stroke is not divided into partial volumes.

8. The dialysis machine according to claim 7, **characterized in that** the first operating condition is set with smaller desired delivery volumes and the second operating conditions is set with comparatively larger desired delivery volumes.

9. The dialysis machine according to claim 8, **characterized in that** the first operating condition is set when the desired delivery volume is not more than one half, one third or one fourth of the maximum delivery volume per stroke of the pump.

10. The dialysis machine according to any of the preceding claims, **characterized in that** the dialysis machine includes a concentrate container for receiving the concentrate and that on its suction side the pump of the device is connected with the concentrate container.

11. A method for delivering concentrate which serves for preparing dialysis fluids in a dialysis machine, wherein the concentrate is delivered through a pump designed as displacement pump,
**characterized in**
**that** during a single suction stroke in a first operating condition the pump sucks in a volume exceeding the desired delivery volume for the purpose of discharging a desired delivery volume and discharges portions of the volume sucked in by performing a plurality of partial delivery strokes in the form of partial volumes which are smaller than the volume sucked in, wherein the partial volumes each correspond to the desired delivery volume.

12. The method according to claim 11, **characterized in that** the volume sucked in during a suction stroke, which exceeds the desired delivery volume, lies in a range of 0.5 to 1.0 of the maximum delivery volume per stroke of the pump.

13. The method according to claim 11 or 12, **characterized in that** the volume sucked in during a suction stroke, which exceeds the desired delivery volume, corresponds to the maximum delivery volume per stroke of the pump.

14. The method according to any of claims 11 to 13, **characterized in that** the partial volumes discharged are identical or substantially identical.

15. The method according to any of claims 11 to 14, **characterized in that** the ratio of the volume sucked in during a suction stroke to a partial volume discharged is about 10:1 or below.

16. The method according to any of claims 11 to 15, **characterized in that** in a second operating condition the pump discharges the volume sucked in during a suction stroke in one delivery stroke.

17. The method according to claim 16 and according to any of claims 11 to 15, **characterized in that** the operating condition of the pump is chosen in dependence on the desired delivery volume.

18. The method according to claim 17, **characterized in that** with smaller desired delivery volumes the pump operates in its first operating condition and with comparatively larger desired delivery volumes in its second operating condition.

19. The method according to claim 18, **characterized in that** the pump operates in its first operating condition when the desired delivery volume is not more than one half, one third or one fourth of the maximum delivery volume per stroke of the pump.

## Revendications

1. Appareil de dialyse avec un dispositif de transport de concentrat qui sert à préparer des liquides de dialyse dans un appareil de dialyse, où le dispositif présente au moins une pompe réalisée comme pompe de refoulement ainsi qu'au moins une unité de commande commandant le fonctionnement de la pompe, **caractérisé en ce que** l'unité de commande spécifie que la pompe, pour la délivrance d'un volume de transport de consigne, dans un premier état de fonctionnement, aspire lors d'une seule course d'aspiration un volume dépassant le volume de transport de consigne, et délivre le volume aspiré par l'exécution de plusieurs courses de transport partielles sous forme de volumes partiels par portions qui sont plus petits que le volume aspiré, où les volumes partiels correspondent à chaque fois au volume de transport de consigne.

2. Appareil de dialyse selon la revendication 1, **caractérisé en ce que** dans le cas de la pompe, il s'agit d'une pompe à membrane ou à piston.

3. Appareil de dialyse selon la revendication 1 ou 2, **caractérisé en ce que** l'unité de commande spécifie que le volume aspiré lors d'une course d'aspiration, dépassant le volume de transport de consigne, se situe dans une plage de 0,5 à 1,0 du volume de transport maximal par course de la pompe.

4. Appareil de dialyse selon l'une des revendications précédentes, **caractérisé en ce que** l'unité de commande spécifie que le volume aspiré lors d'une course d'aspiration, dépassant le volume de transport de consigne, correspond au volume de transport maximal par course de la pompe.

5. Appareil de dialyse selon l'une des revendications précédentes, **caractérisé en ce que** l'unité de commande spécifie que les volumes partiels délivrés sont identiques ou sont sensiblement identiques.

6. Appareil de dialyse selon l'une des revendications précédentes, **caractérisé en ce que** le rapport du volume aspiré lors d'une course d'aspiration et du volume partiel délivré est de 10:1 ou en dessous.

7. Appareil de dialyse selon l'une des revendications précédentes, **caractérisé en ce que** l'unité de commande, en fonction du volume de transport de consigne, spécifie le premier ou un deuxième état de fonctionnement de la pompe lors duquel il n'y a pas de division du volume aspiré lors d'une course d'aspiration en volumes partiels.

8. Appareil de dialyse selon la revendication 7, **caractérisé en ce que** le premier état de fonctionnement est réglé à des volumes de transport de consigne plus petits et le deuxième état de fonctionnement à des volumes de transport de consigne plus grands par rapport à ceux-ci.

9. Appareil de dialyse selon la revendication 8, **caractérisé en ce que** le premier état de fonctionnement est réglé lorsque le volume de transport de consigne représente au maximum la moitié, un tiers ou un quart du volume de transport maximal par course de la pompe.

10. Appareil de dialyse selon l'une des revendications précédentes, **caractérisé en ce que** l'appareil de dialyse présente un contenant de concentrat pour la réception du concentrat, et **en ce que** la pompe du dispositif est en liaison, côté aspiration, avec le contenant de concentrat.

11. Procédé de transport de concentrat, qui sert à la préparation de liquides de dialyse dans un appareil de dialyse, où le concentrat est convoyé par une pompe réalisée comme pompe de refoulement, **caractérisé en ce que** la pompe, dans un premier état de fonctionnement, pour la délivrance d'un volume de transport de consigne, aspire lors d'une seule course d'aspiration un volume dépassant le volume de transport de consigne et délivre le volume aspiré en exécutant plusieurs courses de transport partielles sous forme de volumes partiels par portions qui sont plus petits que le volume aspiré, ou les volumes partiels correspondent à chaque fois au volume de transport de consigne.

12. Procédé selon la revendication 11, **caractérisé en ce que** le volume aspiré lors d'une course d'aspiration, dépassant le volume de transport de consigne, se situe dans une plage de 0,5 à 1,0 du volume de transport maximal par course de la pompe.

13. Procédé selon la revendication 11 ou 12, **caractérisé en ce que** le volume aspiré lors d'une course d'aspiration, dépassant le volume de transport de consigne, correspond au volume de transport maximal par course de la pompe.

14. Procédé selon l'une des revendications 11 à 13, **caractérisé en ce que** les volumes partiels délivrés sont identiques ou sont sensiblement identiques.

15. Procédé selon l'une des revendications 11 à 14, **caractérisé en ce que** le rapport du volume aspiré lors d'une course d'aspiration et du volume partiel délivré est de 10:1 ou en dessous.

16. Procédé selon l'une des revendications 11 à 15, **caractérisé en ce que** la pompe, dans un deuxième état de fonctionnement, délivre le volume aspiré lors d'une course d'aspiration en une course de transport.

17. Procédé selon la revendication 16 ainsi que selon l'une des revendications 11 à 15, **caractérisé en ce que** l'état de fonctionnement de la pompe est sélectionné en fonction du volume de transport de consigne.

18. Procédé selon la revendication 17, **caractérisé en ce que** la pompe, dans le cas de volumes de transport de consigne plus petits, fonctionne dans son premier état de fonctionnement et dans le cas de volumes de transport de consigne plus grands par rapport à ceux-ci, fonctionne dans son deuxième état de fonctionnement.

19. Procédé selon la revendication 18, **caractérisé en ce que** la pompe fonctionne dans son premier état de fonctionnement lorsque le volume de transport de consigne représente au maximum la moitié, un tiers ou un quart du volume de transport maximal par course de la pompe.
